# EUROPEAN PATENT APPLICATION

(11) **EP 4 374 868 A1**
(43) Date of publication of application: **29.05.2024**
(21) Application number: 22845468.2
(22) Date of filing: 22.07.2022
(51) Int. Cl.: A61K 38/00, A61K 38/16, A61K 39/00, C07K 14/485, A61P 17/02, A61P 11/00

(54) **PHARMACEUTICAL COMPOSITION OR DRUG KIT FOR ALLEVIATING OR TREATING FIBROTIC DISEASES, AND USE THEREOF**

(30) Priority: 22.07.2021 CN 202110831690
(71) Applicant: SHANGHAI SYNVIDA BIOTECHNOLOGY CO. LTD., Shanghai 201303 (CN)
(72) Inventor: LIU, Junling, Shanghai 201203 (CN); FAN, Xuemei, Shanghai 201203 (CN); SONG, Haoliang, Shanghai 201203 (CN); LIU, Yanjun, Shanghai 201203 (CN)
(74) Representative: Delbarba, Andrea
(86) International application number: PCT/CN2022/107528
(87) International publication number: WO 2023/001305

(57) **Abstract**

The present invention provides a pharmaceutical composition or drug kit for alleviating or treating fibrotic diseases, and a use thereof. Disclosed is a novel medication regimen in which a platelet endothelial aggregation receptor 1 (PEAR1) agonist and pirfenidone or nintedanib are used in combination.

## Description

This application claims priority to Chinese application No. 202110831690.5 filed on July 22, 2021.

### TECHNICAL FIELD

The disclosure belongs to the field of biomedicine; more specifically, the disclosure relates to a pharmaceutical composition or drug kit for alleviating or treating fibrotic diseases and a use thereof.

### TECHNICAL BACKGROUND

Injury to any organ or tissue triggers complex cellular and molecular reactions, ultimately leading to tissue fibrosis. While such a tissue fibrosis may be characterized by adaptive tissue repair within a short term, an organ or tissue scar will occur when the fibrosis is in a long term progress, eventually resulting in tissue and cell dysfunction and organ failure. Due to known or unknown reasons, fibrosis may occur in most human organs and tissues, such as skin, lungs, liver, heart, kidney, pancreas, spleen, nervous system, and bone marrow, posing a serious threat to human health. Primary pathological changes of fibrosis comprise an increase of fibrous connective tissue within organ tissues, a decrease of parenchymal cells, and continuous progression, leading to structural damage, functional decline, even organ failure, seriously threatening human health and life. Worldwide, tissue fibrosis is a major cause of disability and death in many diseases and nearly 45% of patients who die from various diseases can be attributed to tissue fibro proliferative diseases. Main mechanism of fibrosis in these organs is essentially chartered with fibroblast proliferation and a large amount of extracellular matrix deposition accompanied with inflammatory injuries and destroyed tissue structures.

Pulmonary fibrosis is the most common and serious fibrotic disease, characterized by fibroblast proliferation and the accumulation of a large amount of extracellular matrix, often accompanied with inflammation, tissue damage, and structural disruption. It represents the end-stage changes of a large class of lung diseases where abnormal repair of damaged lung tissue leads to structural abnormalities (scar formation). In most patients, the cause of pulmonary fibrosis is unknown, so it is classified as idiopathic interstitial pneumonia. Among the idiopathic interstitial pneumonia, one type with pulmonary fibrosis as the main manifestation is called idiopathic pulmonary fibrosis (IPF). It is a severe interstitial lung disease that can lead to progressive loss of lung functions, ultimately resulting in respiratory failure and death. Currently, in clinical, only antitumor drugs pirfenidone and nintedanib are used as orphan drugs to treat IPF. By using pirfenidone and nintedanib, the decline of pulmonary functional indicators in IPF patients can be slowed down and the disease process can be alleviated to a certain extent. However, respiratory symptoms cannot be improved.

Therefore, there is an urgent need in this field to develop a drug that can effectively prevent or improve the deterioration of pulmonary fibrosis, restoring normal organ functions in affected tissues.

### SUMMARY OF THE DISCLOSURE

The purpose of present disclosure is to provide a pharmaceutical composition or drug kit for alleviating or treating fibrotic diseases, and a use thereof.

In the first aspect, the present disclosure provides the use of a pharmaceutical combination in the preparation of a mixture, a pharmaceutical composition or a drug kit for alleviating or treating fibrotic diseases; wherein, the pharmaceutical combination comprises: (a) a Pearl (Platelet Endothelial Aggregation Receptor1) gene, an encoded protein or an agonist thereof; and (b) nintedanib or a derivative thereof, pirfenidone or a derivative thereof, or a combination thereof.

In one embodiment, the fibrotic diseases comprise (but are not limited to): pulmonary fibrosis, liver fibrosis, cardiac fibrosis, renal fibrosis, myelofibrosis, scars in skin and/or scars in soft tissues.

In another embodiment, the pulmonary fibrosis is idiopathic pulmonary fibrosis (IPF). Preferably, the pulmonary fibrosis also comprises restrictive lung diseases accompanied with pulmonary fibrosis.

In another embodiment, the cardiac fibrosis is myocardial fibrosis, especially myocardial fibrosis after myocardial infarction.

In another embodiment, the skin fibrosis comprises a disease in which normal cells in skin or soft tissues are replaced by fibroblasts because of traumatic factors or other various reasons.

In another embodiment, the traumatic factors comprise trauma, burns, scars formed during surgery or cosmetic surgery, or keloids formed on the skin in susceptible individuals with cicatricial diathesis, or scleroderma, etc.

In another embodiment, the various reasons comprise one or more of infection, trauma, drug, radiation, foreign matter and hyperimmune.

In another embodiment, the Pearl gene or encoded protein thereof is derived from mammals.

In another embodiment, the Pearl gene or encoded protein thereof is derived from human, mouse, rat, cat, dog, monkey, camel, alpaca or a combination thereof.

In another embodiment, the Pearl gene or encoded protein thereof is derived from human or mouse.

In another embodiment, the Pearl gene, encoded protein or agonist thereof comprises an agent that increase the expression, activity, stability, and/or effective time of Pearl; preferably, it comprises (but are not limited to): an antibody that specifically activates Pearl, an antigen-binding fragment or variant thereof, an expression construct for recombinantly expressing Pearl, a chemical agonist of Pearl, an up-regulator that promotes the driving ability by a promoter of Pearl gene; more preferably, the chemical agonist of Pearl comprise: FcεR1α, dextran sulfate, fucoidan, or combinations thereof.

In another embodiment, the expression constructs (expression vectors) comprise: viral vectors, non-viral vectors; for example, the expression vectors comprise: adeno-associated virus vectors, lentiviral vectors, adenoviral vectors.

In another embodiment, the mixture, pharmaceutical composition or drug kit is also used for inhibiting collagen synthesis by fibroblasts.

In another embodiment, the mixture, pharmaceutical composition or drug kit is also used for inhibiting protein expression of extracellular matrix by fibroblasts.

In another aspect, the present disclosure provides a pharmaceutical composition for alleviating or treating fibrotic diseases, wherein the pharmaceutical composition comprises: (a) a Pearl gene, an encoded protein or an agonist thereof; (b) nintedanib or a derivative thereof, pirfenidone or a derivative thereof, or a combination thereof; and a pharmaceutically acceptable carrier.

In another aspect, the present disclosure provides a mixture for alleviating or treating fibrotic diseases, wherein the mixture is composed of the following components: (a) a Pearl gene, an encoded protein or an agonist thereof; and (b) nintedanib or a derivative thereof, pirfenidone or a derivative thereof, or a combination thereof.

In another aspect, the present disclosure provides a drug kit for alleviating or treating fibrotic diseases, wherein the drug kit comprises a container or package, and separated or mixed in the container or package: (a) a Pearl gene, an encoded protein or an agonist thereof; and (b) nintedanib or a derivative thereof, pirfenidone or a derivative thereof, or a combination thereof.

In another aspect, the present disclosure provides a drug kit for alleviating or treating fibrotic diseases, wherein the drug kit comprises the pharmaceutical composition described above.

In another aspect, the present disclosure provides a drug kit for alleviating or treating fibrotic diseases, wherein the drug kit comprises the mixture described above.

In another embodiment, the derivatives comprise: pharmaceutically acceptable salts, pharmaceutical precursors, isomers, solvates.

In another embodiment, the drug kit also comprises: instructions, illustrating the method of using the contents of the kit to alleviate or treat fibrotic diseases.

In another embodiment, the (a) Pearl gene, an encoded protein or an agonist thereof; and (b) nintedanib or a derivative thereof, pirfenidone or a derivative thereof are administered simultaneously, sequentially or by cross-administration.

In another embodiment, according to the instructions: the Pearl gene, encoded protein or agonist thereof is administered once every 1-4 weeks, and/or the nintedanib or derivatives thereof and/or pirfenidone or derivatives thereof are administered 1-6 times (e.g., 2, 3, 4, 5 times) per day.

In another embodiment, the pharmaceutical composition, mixture or drug kit further comprises one or more of FcεR1α, dextran sulfate, fucoidan, aptamers and small molecule compounds as Pearl agonists; more preferably comprising FcεR1α, dextran sulfate and fucoidan.

In another embodiment, (a) is an antibody that specifically activates Pearl, an antigen-binding fragment or variant thereof; wherein, the mass ratio of the content (content/amount in the mixture, pharmaceutical composition or drug kit) of the antibody that specifically activates Pearl, antigen-binding fragment or variant thereof in (a) and the nintedanib or derivative thereof in (b) is 1:(1~500), preferably 1:(1~300), more preferably 1:(3~200).

In another embodiment, (a) is an antibody that specifically activates Pearl, an antigen-binding fragment or variant thereof; wherein, the mass ratio of the content (content/amount in the mixture, pharmaceutical composition or drug kit) of the antibody that specifically activates Pearl, antigen-binding fragment or variant thereof in (a) and the pirfenidone or derivative thereof in (b) is 1:(5~2000), preferably 1:(5~1000), preferably 1:(5~500), more preferably 1:(10~300).

In another embodiment, the ratio of the content of the antibody that specifically activates Pearl, antigen-binding fragment or variant thereof in (a) and the nintedanib or derivative thereof in (b) is for example, but not limited to 1:400, 1:350, 1:250, 1:200, 1:150, 1:100, 1:80, 1:60, 1:50, 1:30, 1:25, 1:20, 1:15, 1:10, 1:5, 1:2, and so on.

In another embodiment, the ratio of the content of the antibody that specifically activates Pearl, antigen-binding fragment or variant thereof in (a) and the nintedanib or derivative thereof in (b) is for example, but not limited to 1:10, 1:20,1:50, 1:60, 1:120, 1:150, 1:200, 1:250, 1:300, 1:350, 1:400, 1:500, 1:600, 1:800, 1:1200, 1:1800, and so on.

In another embodiment, the agonist is an antibody that specifically activates Pearl, an antigen-binding fragment or variant thereof.

In another embodiment, the antibody comprises a heavy chain variable region and/or a light chain variable region, and the HCDR1, HCDR2 and HCDR3 amino acid sequences of the heavy chain variable region are as shown in SEQ ID NO: 26, 27 and 28, or as shown in SEQ ID NO: 26, 27 and 29, or as shown in SEQ ID NO: 26, 30 and 28, or as shown in SEQ ID NO: 31, 27 and 32, or as shown in SEQ ID NO: 33, 34 and 35; and/or the LCDR1, LCDR2 and LCDR3 amino acid sequences of the light chain variable region are as shown in SEQ ID NO: 45, GAT and SEQ ID NO: 46, or as shown in SEQ ID NO: 47, SAS and SEQ ID NO: 28, or as shown in SEQ ID NO: 49, DTS and SEQ ID NO: 50, or as shown in SEQ ID NO: 51, GAT and SEQ ID NO: 52.

In another embodiment, the heavy chain variable region of the antibody is as shown in SEQ ID NO: 1, 2, 3, 4, 5, 7, 8, 9, 10, 11, 12, 13, 14 or 15, or the light chain variable region of the antibody is as shown in SEQ ID NO: 16, 17, 18, 19, 20, 22, 23, 24 or 25.

In another embodiment, the variant of the antibody has a sequence with an addition, deletion or substitution of one or several, preferably within 1, 2, 3, 4, 5, 6, 7, 8, 9, or 10 amino acid residues in the CDR, FR or full-length sequence.

In another embodiment, the variant has or substantially has the activity of targeting and activating Pearl.

In another embodiment, the HCDR1 of the variant has a mutation from S to A or T at the third position, a mutation from G to D at the sixth position, and/or, a mutation from P to T or Y at the eighth position in the amino acid sequence as shown in SEQ ID NO: 26; and/or, the HCDR2 of the variant has a mutation from P to S or G at the third position, a mutation from Y to N at the fourth position, and/or, a mutation from T to A at the eighth position in the amino acid sequence as shown in SEQ ID NO:27; and/or, the HCDR3 of the variant has a mutation from A to D at the ninth position and/or, a mutation from Y to F at the tenth position in the amino acid sequence as shown in SEQ ID NO:28.

In another embodiment, in the antibody or the variant thereof, HCDR1 comprises any one of the amino acid sequences selected from SEQ ID NO: 26, 31, 33, 36 and SEQ ID NO: 39; HCDR2 comprises any one of the amino acid sequences selected from SEQ ID NO: 27, 30, 34, 37, and SEQ ID NO: 40-44; and, HCDR3 comprises any one of the amino acid sequences selected from SEQ ID NO: 28, 29, 32, 35 and SEQ ID NO: 38; and/or, the LCDR1 comprises any one of the amino acid sequences selected from SEQ ID NO: 45, 47, 49, 51 and SEQ ID NO: 53; LCDR2 comprises any one of the amino acid sequences selected from GAT, SAS, DTS and LVS; and, LCDR3 comprises any one of the amino acid sequences selected from SEQ ID NO: 46, 48, 50, 52 or SEQ ID NO: 54.

In another embodiment, in the antibody or the variant thereof, the HFR (framework region of heavy chain variable region) 1 comprises any one of the amino acid sequences selected from SEQ ID NO: 55, 56 and SEQ ID NO: 57; HFR2 comprises any one of the amino acid sequences selected from SEQ ID NO: 58, 59, 60, 61, 62, 63, 64, 65 and SEQ ID NO: 66; and, HFR3 comprises any one of the amino acid sequences selected from SEQ ID NO: 67 , 68, 69, 70, 71, 72, 73, 74, 75 and SEQ ID NO: 76; and HFR4 comprises any one of the amino acid sequences selected from SEQ ID NO: 77 and SEQ ID NO: 78.

In another embodiment, in the antibody or the variant thereof, the LFR (framework region of light chain variable region) 1 comprises any one of the amino acid sequences selected from SEQ ID NO: 79, 80,81, 82, 83 and SEQ ID NO: 84; LFR2 comprises any one of the amino acid sequences selected from SEQ ID NO: 85, 86, 87, 88, 89 and SEQ ID NO: 90; and LFR3 comprises any one of the amino acid sequences selected from SEQ ID NO: 91 , 92, 93, 94, 95, 96, 97 and SEQ ID NO: 98; and LFR4 comprises any one of the amino acid sequences selected from SEQ ID NO: 99, 100, 101, 102 and SEQ ID NO: 103.

In another embodiment, the antibody or variant thereof comprises the amino acid sequences respectively:
HCDR1, HCDR2 and HCDR3 as shown in SEQ ID NO: 26, SEQ ID NO: 27, SEQ ID NO: 28, LCDR1, LCDR2 and LCDR3 as shown in SEQ ID NO: 45, GAT, SEQ ID NO: 46; or,
HCDR1, HCDR2 and HCDR3 as shown in SEQ ID NO: 26, SEQ ID NO: 27, SEQ ID NO: 29, LCDR1, LCDR2 and LCDR3 as shown in SEQ ID NO: 45, GAT, SEQ ID NO: 46; or,
HCDR1, HCDR2 and HCDR3 as shown in SEQ ID NO: 26, SEQ ID NO: 30, SEQ ID NO: 28, LCDR1, LCDR2 and LCDR3 as shown in SEQ ID NO: 47, GTS, SEQ ID NO: 48; or,
HCDR1, HCDR2 and HCDR3 as shown in SEQ ID NO: 26, SEQ ID NO: 27, SEQ ID NO: 29, LCDR1, LCDR2 and LCDR3 as shown in SEQ ID NO: 49, DTS, SEQ ID NO: 50; or,
HCDR1, HCDR2 and HCDR3 as shown in SEQ ID NO: 26, SEQ ID NO: 40, SEQ ID NO: 28, LCDR1, LCDR2 and LCDR3 as shown in SEQ ID NO: 49, GAT, SEQ ID NO: 54; or,
HCDR1, HCDR2 and HCDR3 as shown in SEQ ID NO: 26, SEQ ID NO: 41, SEQ ID NO: 28, LCDR1, LCDR2 and LCDR3 as shown in SEQ ID NO: 49, SAS, SEQ ID NO: 54; or,
HCDR1, HCDR2 and HCDR3 as shown in SEQ ID NO: 26, SEQ ID NO: 42, SEQ ID NO: 28, LCDR1, LCDR2 and LCDR3 as shown in SEQ ID NO: 49, LVS, SEQ ID NO: 52; or,
HCDR1, HCDR2 and HCDR3 as shown in SEQ ID NO: 26, SEQ ID NO: 43, SEQ ID NO: 32, LCDR1, LCDR2 and LCDR3 as shown in SEQ ID NO: 45, GAT, SEQ ID NO: 54; or,
HCDR1, HCDR2 and HCDR3 as shown in SEQ ID NO: 31, SEQ ID NO: 27, SEQ ID NO: 32, LCDR1, LCDR2 and LCDR3 as shown in SEQ ID NO: 51, GAT, SEQ ID NO: 52; or,
HCDR1, HCDR2 and HCDR3 as shown in SEQ ID NO: 31, SEQ ID NO: 37, SEQ ID NO: 32, LCDR1, LCDR2 and LCDR3 as shown in SEQ ID NO: 53, LVS, SEQ ID NO: 46; or,
HCDR1, HCDR2 and HCDR3 as shown in SEQ ID NO: 31, SEQ ID NO: 34, SEQ ID NO: 32, LCDR1, LCDR2 and LCDR3 as shown in SEQ ID NO: 45, SAS, SEQ ID NO: 50; or,
HCDR1, HCDR2 and HCDR3 as shown in SEQ ID NO: 31, SEQ ID NO: 30, SEQ ID NO: 35, LCDR1, LCDR2 and LCDR3 as shown in SEQ ID NO: 47, GAT, SEQ ID NO: 50; or,
HCDR1, HCDR2 and HCDR3 as shown in SEQ ID NO: 31, SEQ ID NO: 42, SEQ ID NO: 28, LCDR1, LCDR2 and LCDR3 as shown in SEQ ID NO: 49, DTS, SEQ ID NO: 54; or,
HCDR1, HCDR2 and HCDR3 as shown in SEQ ID NO: 33, SEQ ID NO: 34, SEQ ID NO: 35, LCDR1, LCDR2 and LCDR3 as shown in SEQ ID NO: 45, GAT, SEQ ID NO: 46; or,
HCDR1, HCDR2 and HCDR3 as shown in SEQ ID NO: 33, SEQ ID NO: 37, SEQ ID NO: 32, LCDR1, LCDR2 and LCDR3 as shown in SEQ ID NO: 53, LVS, SEQ ID NO: 46; or,
HCDR1, HCDR2 and HCDR3 as shown in SEQ ID NO: 33, SEQ ID NO: 30, SEQ ID NO: 35, LCDR1, LCDR2 and LCDR3 as shown in SEQ ID NO: 47, GAT, SEQ ID NO: 50; or,
HCDR1, HCDR2 and HCDR3 as shown in SEQ ID NO: 33, SEQ ID NO: 42, SEQ ID NO: 28, LCDR1, LCDR2 and LCDR3 as shown in SEQ ID NO: 49, DTS, SEQ ID NO: 54; or,
HCDR1, HCDR2 and HCDR3 as shown in SEQ ID NO: 36, SEQ ID NO: 37, SEQ ID NO: 38, LCDR1, LCDR2 and LCDR3 as shown in SEQ ID NO: 53, LVS, SEQ ID NO: 54; or,
HCDR1, HCDR2 and HCDR3 as shown in SEQ ID NO: 36, SEQ ID NO: 34, SEQ ID NO: 32, LCDR1, LCDR2 and LCDR3 as shown in SEQ ID NO: 45, SAS, SEQ ID NO: 50; or,
HCDR1, HCDR2 and HCDR3 as shown in SEQ ID NO: 36, SEQ ID NO: 30, SEQ ID NO: 35, LCDR1, LCDR2 and LCDR3 as shown in SEQ ID NO: 47, GAT, SEQ ID NO: 50; or,
HCDR1, HCDR2 and HCDR3 as shown in SEQ ID NO: 36, SEQ ID NO: 42, SEQ ID NO: 28, LCDR1, LCDR2 and LCDR3 as shown in SEQ ID NO: 49, DTS, SEQ ID NO: 54; or,
HCDR1, HCDR2 and HCDR3 as shown in SEQ ID NO: 39, SEQ ID NO: 27, SEQ ID NO: 28, LCDR1, LCDR2 and LCDR3 as shown in SEQ ID NO: 45, GAT, SEQ ID NO: 46; or,
HCDR1, HCDR2 and HCDR3 as shown in SEQ ID NO: 39, SEQ ID NO: 32, LCDR1, LCDR2 and LCDR3 as shown in SEQ ID NO: 45, SAS, SEQ ID NO: 50; or,
HCDR1, HCDR2 and HCDR3 as shown in SEQ ID NO: 39, SEQ ID NO: 30, SEQ ID NO: 35, LCDR1, LCDR2 and LCDR3 as shown in SEQ ID NO: 47, GAT, SEQ ID NO: 50; or,
HCDR1, HCDR2 and HCDR3 as shown in SEQ ID NO: 39, SEQ ID NO: 42, SEQ ID NO: 28, LCDR1, LCDR2 and LCDR3 as shown in SEQ ID NO: 49, DTS, SEQ ID NO: 54; or,
HCDR1, HCDR2 and HCDR3 as shown in SEQ ID NO: 39, SEQ ID NO: 37, SEQ ID NO: 28, LCDR1, LCDR2 and LCDR3 as shown in SEQ ID NO: 49, GAT, SEQ ID NO: 48; or,
HCDR1, HCDR2 and HCDR3 as shown in SEQ ID NO: 39, SEQ ID NO: 40, SEQ ID NO: 28, LCDR1, LCDR2 and LCDR3 as shown in SEQ ID NO: 45, GAT, SEQ ID NO: 46; or,
HCDR1, HCDR2 and HCDR3 as shown in SEQ ID NO: 39, SEQ ID NO: 41, SEQ ID NO: 28, LCDR1, LCDR2 and LCDR3 as shown in SEQ ID NO: 45, GAT, SEQ ID NO: 46; or,
HCDR1, HCDR2 and HCDR3 as shown in SEQ ID NO: 39, SEQ ID NO: 42, SEQ ID NO: 28, LCDR1, LCDR2 and LCDR3 as shown in SEQ ID NO: 45, GAT, SEQ ID NO: 46; or,
HCDR1, HCDR2 and HCDR3 as shown in SEQ ID NO: 39, SEQ ID NO: 43, SEQ ID NO: 28, LCDR1, LCDR2 and LCDR3 as shown in SEQ ID NO: 45, GAT, SEQ ID NO: 46; or,
HCDR1, HCDR2 and HCDR3 as shown in SEQ ID NO: 39, SEQ ID NO: 44, SEQ ID NO: 28, LCDR1, LCDR2 and LCDR3 as shown in SEQ ID NO: 45, GAT, SEQ ID NO: 46.

In another embodiment, the amino acid sequences of the light chain variable region and the heavy chain variable region are respectively shown in SEQ ID NO: 16 and SEQ ID NO: 1; or, the amino acid sequences of the light chain variable region and the heavy chain variable region are respectively are shown in SEQ ID NO: 16 and SEQ ID NO: 2; or, the amino acid sequences of the light chain variable regions and the heavy chain variable regions are respectively shown in SEQ ID NO: 17 and SEQ ID NO: 3; or, the amino acid sequences of the light chain variable region and the heavy chain variable region are respectively shown in SEQ ID NO: 18 and SEQ ID NO: 2; or, the amino acid sequences of the light chain variable region and the heavy chain variable region are respectively shown in SEQ ID NO: 19 and SEQ ID NO: 4; or, the amino acid sequences of the light chain variable region and the heavy chain variable region are respectively shown in SEQ ID NO: 20 and SEQ ID NO: 5; or, the amino acid sequences of the light chain variable region and the heavy chain variable region are respectively shown in SEQ ID NO: 21 and SEQ ID NO: 6; or, the amino acid sequences of the light chain variable region and the heavy chain variable region are respectively shown in SEQ ID NO: 22 and SEQ ID NO: 7; or, the amino acid sequences of the light chain variable region and the heavy chain variable region are respectively shown in SEQ ID NO : 23 and SEQ ID NO: 7; or, the amino acid sequences of the light chain variable region and the heavy chain variable region are respectively shown in SEQ ID NO: 24 and SEQ ID NO: 7; or, the amino acid sequences of the light chain variable region and the heavy chain variable region are respectively shown in SEQ ID NO: 25 and SEQ ID NO: 7; or, the amino acid sequences of the light chain variable region and the heavy chain variable region are respectively shown in SEQ ID NO: ID NO: 22 and SEQ ID NO: 8; or, the amino acid sequences of the light chain variable region and the heavy chain variable region are respectively shown in SEQ ID NO: 23 and SEQ ID NO: 8; or, the amino acid sequences of the light chain variable region and the heavy chain variable region are respectively shown in SEQ ID NO: 24 and SEQ ID NO: 8; or, the amino acid sequences of the light chain variable region and the heavy chain variable region are respectively shown in SEQ ID NO: 25 and SEQ ID NO: 8; or, the amino acid sequences of the light chain variable region and the heavy chain variable region are respectively shown in SEQ ID NO: 22 and SEQ ID NO: 9; or, the amino acid sequences of the light chain variable region and the heavy chain variable region are respectively shown in SEQ ID NO: 23 and SEQ ID NO: 9; or, the amino acid sequences of the light chain variable region and the heavy chain variable region are respectively shown in SEQ ID NO: 24 and SEQ ID NO: 9; or, the amino acid sequences of the light chain variable region and the heavy chain variable region are respectively shown in SEQ ID NO: 25 and SEQ ID NO: 9 or, the amino acid sequences of the light chain variable region and the heavy chain variable region are respectively shown in SEQ ID NO: 22 and SEQ ID NO: 10; or, the amino acid sequences of the light chain variable region and the heavy chain variable region are respectively shown in SEQ ID NO: 23 and SEQ ID NO: 10; or, the amino acid sequences of the light chain variable region and the heavy chain variable region are respectively shown in SEQ ID NO: 24 and SEQ ID NO: 10; or, the amino acid sequences of the light chain variable region and the heavy chain variable region are respectively shown in SEQ ID NO: 25 and SEQ ID NO: 10; or, the amino acid sequences of the light chain variable region and the heavy chain variable region are respectively shown in SEQ ID NO: 22 and SEQ ID NO: 11 or, the amino acid sequences of the light chain variable region and the heavy chain variable region are respectively shown in SEQ ID NO: 22 and SEQ ID NO: 12; or, the amino acid sequences of the light chain variable region and the heavy chain variable region are respectively shown in SEQ ID NO: 22 and SEQ ID NO: 13 respectively; or, the amino acid sequences of the light chain variable region and the heavy chain variable region are respectively shown in SEQ ID NO: 22 and SEQ ID NO: 14; or, the amino acid sequences of the light chain variable region and the heavy chain variable region are respectively shown in SEQ ID NO: 22 and SEQ ID NO: 15.

In another embodiment, the variant is 80%, 85%, 90%, 95%, 96%, 97%, 98%, or 99% identical to the amino acid sequence of the antibody.

In another embodiment, the antibody is a full-length antibody, Fab, Fab', F(ab')₂, Fv such as scFv, bispecific antibody, multispecific antibody, or single domain antibody, or it is derived from the above-mentioned monoclonal or polyclonal antibodies produced by the antibody.

In another embodiment, the antibody or variant thereof is a full-length antibody, and the heavy chain constant region of the full-length antibody is selected from heavy chain constant regions of hIgG1, hIgG2, hIgG3 or hIgG4 or a derivative sequence thereof.

In another embodiment, the light chain constant region of the antibody is selected from κ chain or λ chain or a derivative sequence thereof.

In another embodiment, the derivative sequence of the heavy chain constant region comprises a sequence of heavy chain constant region of hIgG1, hIgG2, hIgG3 or hIgG4 with an addition, deletion or substitution of one or several, preferably within 1, 2, 3, 4, 5, 6, 7, 8, 9, or 10 amino acid residues.

In another embodiment, the derivative sequence of the heavy chain constant region is 80%, 85%, 90%, 95%, 96%, 97%, 98%, or 99% identical to the amino acid sequence of the heavy chain constant region of hIgG1, hIgG2, hIgG3 or hIgG4.

In another embodiment, the heavy chain constant region of the full-length antibody is the heavy chain constant region of hIgG3 or hIgG4 or a derivative sequence thereof.

In another embodiment, the derivative sequence is an amino acid sequence after 1-2 amino acid substitutions at the heavy chain constant region of hIgG1, hIgG2, hIgG3 or hIgG4.

In another embodiment, the derivative sequence has a mutation of S228P in hIgG4.

In another embodiment, the light chain constant region is human κ chain.

In another embodiment, in the mixture, pharmaceutical composition or drug kit, the formulation of component, mixture or composition may (based on physical form) comprise formulation selected from gas formulations such as aerosols, dry powder inhalers, aerosol inhalers; liquid formulations such as syrups, injections, suspensions; solid formulations such as lyophilized agents, powders, granules, tablets; semi-solid formulations such as ointments, pastes; or combinations thereof.

In another embodiment, in the mixture, pharmaceutical composition or drug kit, the formulation of component, mixture or composition may be classified based on administration routes, comprising a formulation selected from inhalation formulations for respiratory delivery (such as aerosols, dry powder inhalers, aerosol inhalers), gastrointestinal formulations (such as decoctions, mixtures, granules, pills, tablets, etc.) , rectal formulations (such as suppositories, enemas), injectable formulations (such as intravenous, intramuscular, subcutaneous, intradermal, and acupoint injections), transdermal formulations (such as lotions, coatings, ointments, transdermal plasters, etc.), mucosal formulations (such as eyedrops, oral films, sublingual tablets, mouthwashes), or combinations thereof.

In another embodiment, the antibody specifically activating Pearl is an inhalation formulation, preferably an aerosol and/or an aerosol inhaler.

In another embodiment, the pirfenidone and/or nintedanib are oral formulation, injectable formulation and/or inhalation formulation.

In another embodiment, the pirfenidone and/or nintedanib are oral formulation and/or dry powder inhaler.

In another embodiment, in the mixture, pharmaceutical composition or drug kit, the component, mixture or composition can be administered by inhalation, pulmonary nebulization, local injection, transdermal absorption, subcutaneous injection, local application.

In another aspect, the present disclosure provides a method for alleviating or treating fibrotic diseases, comprising administering a pharmaceutical combination to a subject in need of alleviating or treating, wherein the pharmaceutical combination comprises: (a) a Pearl gene, an encoded protein or an agonist thereof; and (b) nintedanib or a derivative thereof, pirfenidone or a derivative thereof, or a combination thereof.

In another embodiment, in the pharmaceutical combination, (a) and (b) are administered simultaneously or separately; preferably, (a) and (b) are administered separately; more preferably, wherein (a) is administered by inhalation via respiratory delivery, (b) is administered via gastrointestinal delivery.

It should be understood that within the scope of the present disclosure, the above-mentioned technical features of the present disclosure and the technical features specifically described in the following (such as the examples) can be combined with each other to form a preferred technical embodiment. These combined embodiments are also included in the present disclosure.

### DESCRIPTION OF FIGURES

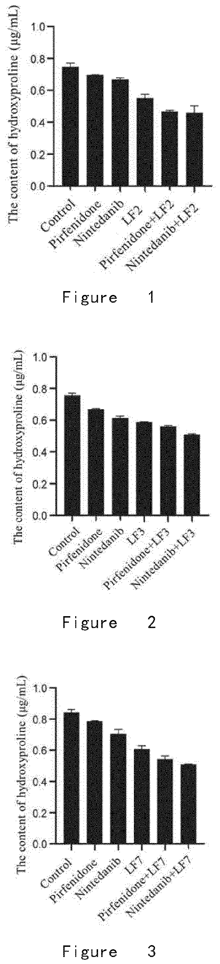

Figure 1. Effects of LF2 antibody alone and in combination with pirfenidone or nintedanib on collagen secretion in fibroblasts. IPF fibroblasts induced by TGFβ were treated with LF2 antibody, pirfenidone, nintedanib, LF2 antibody in combination with pirfenidone, and LF2 antibody in combination with nintedanib for 48 hours. The content of hydroxyproline in cell supernatant was measured using a kit.
Figure 2. Effects of LF3 antibody alone and in combination with pirfenidone or nintedanib on collagen secretion in fibroblasts. IPF fibroblasts induced by TGFβ were treated with LF3 antibody, pirfenidone, nintedanib, LF3 antibody in combination with pirfenidone, and LF3 antibody in combination with nintedanib for 48 hours. The content of hydroxyproline in cell supernatant was measured using a kit.
Figure 3. Effects of LF7 antibody alone and in combination with pirfenidone or nintedanib on collagen secretion in fibroblasts. IPF fibroblasts induced by TGFβ were treated with LF7 antibody, pirfenidone, nintedanib, LF7 antibody in combination with pirfenidone, and LF7 antibody in combination with nintedanib for 48 hours. The content of hydroxyproline in cell supernatant was measured using a kit.

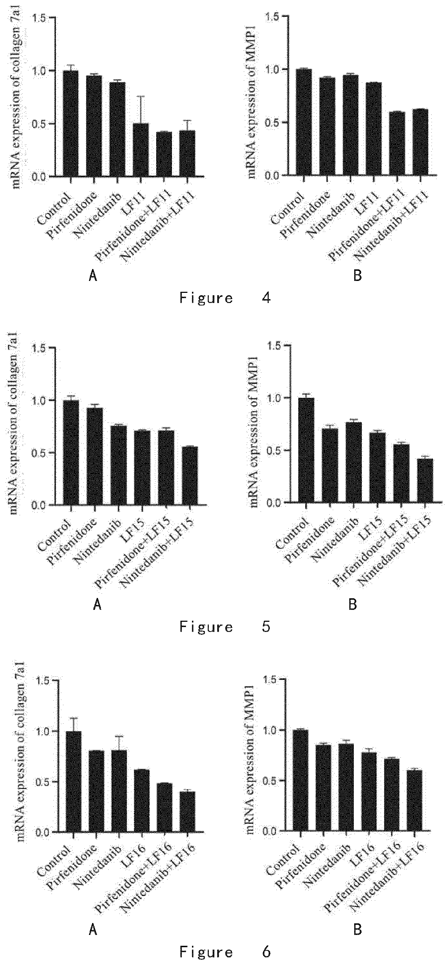

Figure 4. Effects of LF11 antibody alone and in combination with pirfenidone or nintedanib on extracellular matrix synthesis in fibroblasts. Figure 4A-B shows that IPF fibroblasts induced by TGFβ were treated with LF11 antibody, pirfenidone, nintedanib, LF11 antibody in combination with pirfenidone, and LF11 antibody in combination with nintedanib for 48 hours, then were collected for detecting the effects on the synthesis of extracellular matrix protein collagen 7a1 and inhibitors of matrix metalloproteinases by qPCR.
Figure 5. Effects of LF15 antibody alone and in combination with pirfenidone or nintedanib on extracellular matrix synthesis in fibroblasts. Figure 5A-B shows that IPF fibroblasts induced by TGFβ were treated with LF15 antibody, pirfenidone, nintedanib, LF15 antibody in combination with pirfenidone, and LF15 antibody in combination with nintedanib for 48 hours, then were collected for detecting the effects on the synthesis of extracellular matrix protein collagen 7a1 and inhibitors of matrix metalloproteinases by qPCR.
Figure 6. Effects of LF16 antibody alone and in combination with pirfenidone or nintedanib on extracellular matrix synthesis in fibroblasts. Figure 6A-B shows that IPF fibroblasts induced by TGFβ were treated with LF16 antibody, pirfenidone, nintedanib, LF16 antibody in combination with pirfenidone, and LF16 antibody in combination with nintedanib for 48 hours, then were collected for detecting the effects on the synthesis of extracellular matrix protein collagen 7a1 and inhibitors of matrix metalloproteinases by qPCR.

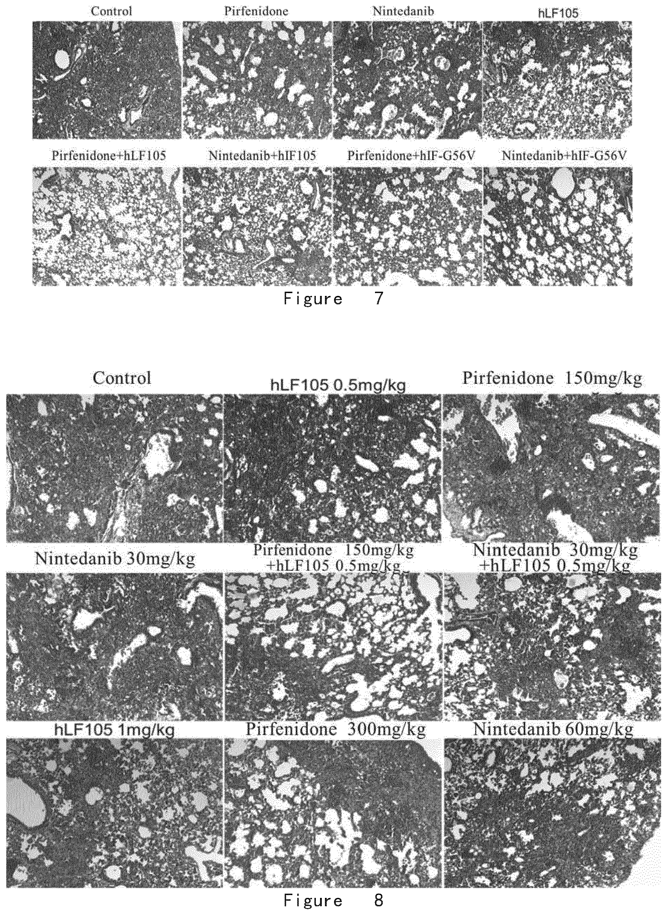

Figure 7. Effects of antibodies in combination with pirfenidone or nintedanib on bleomycin-induced pulmonary fibrosis in mice. Bleomycin-induced pulmonary fibrosis were treated by 1mg/kg hLF105 and hLF-G56V antibodies in combination with 300 mg/kg pirfenidone or 60 mg/kg nintedanib, respectively. Representative images showed results of masson staining on lung histopathological sections in mice. Green represents collagen deposition.
Figure 8. Effects of low-dose hLF105 antibody in combination with low-dose pirfenidone or nintedanib on bleomycin-induced pulmonary fibrosis in mice. Bleomycin-induced pulmonary fibrosis were treated by 0.5mg/kg hLF105 antibody in combination with 150mg/kg pirfenidone or 30mg/kg nintedanib, respectively. Representative images showed results of masson staining on lung histopathological sections in mice. Green represents collagen deposition.

### DETAILED DESCRIPTION

After extensive and in-depth researches, the present inventors unexpectedly discovered that gene and protein expression of Pearl in fibroblasts significantly reduces the activation of fibroblasts. Furthermore, the inventors found that when a Pearl agonist (such as an antibody specifically activating Pearl, an agonistic antibody) is used in combination with pirfenidone or nintedanib, extracellular matrix secreted by fibroblasts reduced significantly, with the symptoms of respiratory system relieved. That is, the combined effect is significantly superior to the individual use of each drug, and the dose of each drug used in combination is significantly lower than that of each drug used individually. Therefore, the combination of the Pearl agonist with nintedanib or pirfenidone can achieve a clinically superior effect and reduce the toxic side effects of nintedanib and pirfenidone.

The inventors have previously applied for patents, using Pearl as a target of fibrotic diseases, constructing an antibody that enhances the activity of Pearl, inhibiting the excessive activation of fibroblasts, so as to achieve the purpose of treating fibrotic diseases. These applications comprise: Chinese patent application 202010076729.2 with a filing date of 2020/1/23 and Chinese patent application 202011212639.8 with a filing date of 2020/11/3. The above-mentioned Chinese patent applications were incorporated in the present disclosure in its entirety. In the present disclosure, a combination scheme of molecules based on Pearl as a regulatory target and other drugs is further proposed.

### Terms

As used herein, the term "treatment (treatment and treating)" comprise the administration of one or more active ingredients to prevent or delay the onset of the symptoms or complications, and to prevent or delay the development of the disease, condition or disorder, and/or to eliminate or control the disease, condition or disorder and alleviate symptoms or complications associated with the disease, condition or disorder. The term "treatment" encompasses both therapeutic, i.e. curative and/or palliative treatment and prophylactic, i.e. prophylactic treatment. Therapeutic treatment refers to the treatment of a patient who has developed one or more of the manifested acute or chronic disorders. Therapeutic treatment may be symptomatic to alleviate the symptoms of a particular indication, or etiological to reverse or partially reverse the symptoms of the indication or to arrest or slow the progression of the disease. Prophylactic treatment ("prophylaxis") refers to therapeutic treatments of a patient at risk of developing one or more symptoms before the clinical onset of the disease, with the aim of reducing the risks.

As used herein, the term "comprise" means that various components can be used together in the mixture or composition of the present disclosure. Therefore, the term "mainly consist of......" and "consist of......" is included in the term "comprise". The term "mainly consist of......" and "consist of......" is included in the term "comprise".

As used herein, "pharmaceutically acceptable" is used herein to refer to components, materials, compositions, and/or formulations that, within the scope of reasonable medical judgment, are suitable for contacting with human and animal tissues without undue toxicity, irritation, allergic reactions, or other problems or complications, accompanied with a reasonable balance between benefits and risks.

As used herein, "effective amount" means the amount of pharmaceutically active component in the present disclosure that (i) treats or prevents the particular disease or condition, (ii) attenuates, improves or eliminates one or more symptoms of the particular disease or disorder, or (iii) prevents or delays the onset of one or more symptoms of the particular disease or disorder described herein. The terms "effective amount", "safe and effective amount" or "therapeutically effective amount" are used interchangeably herein. Those skilled in the art (such as experienced clinicians) can adjust the "safe and effective dose" according to the subj ect.

As used herein, the term "enhance", "promote", and "improve", "increase" are used interchangeably; preferably statistically significantly "increase", "promote", "improve" or "enhance", such as an increase of 2% or higher, 5% or higher, 10% or higher, 15% or higher, 20% or higher, 30% or higher, 50% or higher, 80% or higher, 100% or higher, 200% or higher, 500% or higher.

As used herein, the term "reduce", "decrease", "weaken" are used interchangeably; preferably statistically significantly "inhibit", "reduce", "decrease" or "weaken", such as an decrease of 2% or lower, 5% or lower, 10% or lower, 15% or lower, 20% or lower, 30% or lower, 50% or lower, 80% or lower, 100% or lower, 200% or lower, 500% or lower.

As used herein, "active ingredient" or "pharmaceutically active ingredient" comprises an ingredient selected from: Pearl gene, an encoded protein or an agonist thereof; nintedanib or a derivative thereof; pirfenidone or a derivative thereof.

As used herein, the "derivative" of nintedanib or pirfenidone refers to a compound that substantially retains the function of nintedanib or pirfenidone with certain changes in chemical structure; preferably, the parent nucleus structure of derivative is identical, substantially identical or similar to nintedanib or pirfenidone.

As used herein, the term "antibody or variant thereof' refers to a protein, antibody fragment or polypeptide sequence derived from an immunoglobulin molecule that specifically binds to an antigen. The antibody may be polyclonal or monoclonal, multi-stranded or single-stranded, or an intact immunoglobulin, and may be derived from natural sources or recombinant sources. In a specific embodiment, an "antibody or variant" of the disclosure is isolated and derived from recombinant sources.

As used herein, the term "antibody fragment" refers to at least a portion of the antibody that retains the ability to specifically interact with an antigen (e.g., by binding, steric hindrance, stabilization/destabilization, spatial distribution). Examples of antibody fragments include, but are not limited to, Fab, Fab', F(ab')2, Fv fragment, scFv antibody fragment, disulfide bond-linked Fvs (sdFV), Fd fragment consisting of VH and CH1 domains, linear antibodies, single domain antibodies, multi-specific antibodies formed by antibody fragments (e.g., bivalent fragments of two Fab fragments linked by disulfide bonds in a hinge region) and isolated CDRs or other epitope binding fragments. The antigen-binding fragment can also be incorporated into a single-domain antibody, a maximum antibody, a micro antibody, a nano-antibody, an intracellular antibody, a bi-specific antibody and a chimeric antibody.

Scope: In the entire disclosure, various aspects of the disclosure may be present in the form of ranges. It should be understood that the description in the form of ranges is for convenience and brevity and should not be construed as limiting the scope of the disclosure. Accordingly, the description of the ranges should be considered to specifically disclose all possible sub-ranges and individual numerical values within that range. For example, a description of a range, such as from 1 to 6, should be considered to specifically disclose sub-ranges such as 1 to 3, 1 to 4, 1 to 5, 2 to 4, 2 to 6, 3 to 6, etc. as well as individual numerical values within that range, such as 1, 2, 2.7, 3, 4, 5, 5.3, and 6. As another example, a range, such as having an identity of 95-99%, includes a range of identity of 95%, 96%, 97%, 98% or 99% including sub-ranges such as an identity of 96-99%, 96-98%, 96-97%, 97-99%, 97-98% and 98-99%. This is applicable regardless of the width of the range.

### Fibrotic Diseases

As used herein, the term "fibrotic disease" refers to a disease that results from over or abnormal activation of fibroblasts due to damages to normal tissues or cells, tissue or organ repair initiated by fibroblasts, resulted epithelial-mesenchymal transition of fibroblasts, increased extracellular matrix synthesis, scar tissue substitutions of normal tissues and function loss of normal tissues.

Generally, since fibroblasts are widely distributed in various normal tissues, as long as normal tissue cells are damaged, excessive or abnormal activation of fibroblasts can be induced; such as significant pulmonary fibrosis, hepatic fibrosis or renal fibrosis after lung, liver or kidney inflammation; or, such as skin, muscle or connective tissue proliferative scars caused by surgery, trauma or the like; or, myocardial fibrosis after myocardial infarction and myelofibrosis associated with myeloproliferative diseases, etc. It is indicated that a "fibrotic disease" is a "scar" which spreads over the organs or tissues of the whole body and is caused by over or abnormal activation of fibroblasts due to damaged normal tissues, and the scar directly limits the original normal functions of organs and tissues.

From the mechanism perspective, the fibrotic disease is characterized by fibroblasts (FB) migration to the wound and/or proliferation and massive extracellular matrix aggregation accompanied by inflammation injuries and tissue structure damages. Therefore, FBs play a very important role in the course of fibrosis diseases. FBs are the most leading cell components in connective tissues, and are differentiated from mesenchymal cells in an embryo period. Fibroblast can also exist as a form of fibrocyte, which is in its mature and quiescence status. Fibroblasts and fibrocytes can be converted to each other under certain conditions. In addition, it is reported that FBs can be turned from epithelial cells, macrophages, blood mononuclear cells, endothelial cells and other normal cells through epithelial-mesenchymal transition (epithelial-mesenchymal transition, EMT), and these trans-differentiation processes may be one of the important generation modes of FBs in fibrotic diseases.

Under physiological conditions, FBs synthesize and release extracellular matrix (ECM) components to form tissues, maintain organ morphology and physiological functions, and get employed in repairs after tissue damages. Under pathological conditions, FBs can be transformed into activated fibroblasts by abnormal proliferations, and synthesize and secrete massive ECM proteins; thereby inducing excessive connective tissues and organ fibrosis. In addition, ECM level is further regulated and controlled by the balance between its decomposition enzyme, namely matrix metalloproteinase (MMP) and matrix metalloproteinase tissue inhibitor (TIMPS). In the physiological condition, the synthesis and decomposition of the ECMs in the lung are in a balance; while during the pulmonary fibrosis process, the balance between MMPs/TIMPs system is destroyed, and ECMs is over-accumulated to form a scar structure, which is a key factor in the development of fibrosis. It indicates that FBs, as well as the balance of the MMPs/TIMPS play an important role in fibrotic diseases.

Pulmonary fibrosis is the most common and the most serious fibrosis disease, and refers to pulmonary diseases caused by abnormal activation of fibroblasts and scar formation caused by abnormal repair of alveolar tissue damage. The pulmonary fibrosis, which is mainly characterized by dry cough and progressive dyspnea, seriously deteriorates the respiratory function of the human body. The lung injury aggravates with the development of the disease and the respiratory function of the patient keeps decreasing. The main causes of death comprise the acute exacerbation of the respiratory function and the associated complications thereof.

In most patients, the cause of pulmonary fibrosis is unknown and is classified as idiopathic interstitial pneumonia. The type of disease characterized as pulmonary fibrosis among idiopathic interstitial pneumonia is referred as idiopathic pulmonary fibrosis (IPF). The continuous diffusive alveolar inflammation and alveolar structure disorder of IPF lead to pulmonary interstitial fibrosis to form permanent scars, and the lung function declines irreversibly. Finally, it leads to respiratory failure and causes death of patients. The cause of IPF is not clear, and is currently considered to be related to environmental factors or the use of chemotherapeutic drugs. It may also be an acute attack related to repeated infections caused by viruses, bacteria, fungi, parasites and so on. In addition, autoimmune diseases such as lupus erythematosus and other autoimmune diseases are one of the causes of the disease. The prognosis of IPF is poor, the median survival time of a patient is 2-3 years, the 5-year survival rate is lower than 30%, and therefore, it is referred as a "tumor-like disease".

Since IPF progresses unpredictably, it is quite tough to treat. The treatments to IPF mainly comprise non-drug therapy, including smoking cessation, oxygen therapy, mechanical ventilation, palliative treatment and lung rehabilitation training, etc.; and drug therapy, including hormone therapy, acid-suppression therapy, N-acetylcysteine treatment and the like. Although glucocorticoids have been used for treating pulmonary fibrosis over 50 years, the latest research proves that they are only effective in 20% of patients, and long-term use of glucocorticoids may cause severe adverse reactions. In addition, Meta-analysis of research results showed that single use of N-acetylcysteine treatment cannot reduce the patient's disease death rate, or improve the change value of forced vital capacity (FVC) or the life quality.

Hepatic fibrosis is a pathological process caused by abnormal repair of liver injury. Abnormal proliferation of stellate cells in connective tissues (i.e., liver fibroblasts) and a large amount of secreted extracellular matrix caused by liver injuries are the main causes. Long-term hepatic fibrosis can develop into liver cirrhosis, portal vein high pressure, liver cancer and so on.

Myocardial fibrosis refers to scars formed by abnormal proliferation of myocardial fibroblasts and huge amounts of extracellular matrix induced by myocardial cell necrosis. Myocardial fibrosis will result in heart stiffness, heart dilation, and induce arrhythmia, heart failure and so on.

Renal fibrosis refers to scars formed by abnormal deposition of kidney connective tissue fibroblast extracellular matrix (ECM) due to damages of kidney cells caused by trauma, infection, blood circulation disorder, immune-inflammatory response and etc., and the resulting renal parenchyma sclerosis and function loss.

Bone marrow fibrosis is a disease caused by abnormal deposition of extracellular matrix (ECM) of bone marrow fiber tissues, and is a type of myeloproliferative disease seriously affecting hematopoietic function.

Skin or soft tissue scar formation is caused by huge amounts of ECM produced and deposited by fibroblasts migrated from skin connective tissues to wounded sites of skin or soft tissues. Abnormal scar formation may hinder the healing of chronic wounds and cause fibrosis. Generally, all causes of skin or soft tissue damage, such as surgeries, trauma, empyrosis, local or diffusive inflammation (i.e. autoimmune diseases, scleroderma, etc.) can result in abnormal scars, or excessive connective tissues of soft tissues.

### Pearl gene, a protein or agonist thereof

Pearl, Platelet Endothelial Aggregation Receptor 1 (Gene ID: 375033), is a 150 kDa type I transmembrane protein, and is a member of a Multiple Epidermal Growth Factor-Like domain Receptor family (MEGF) Protein family. Human Pearl contains 1037 amino acids (aa), wherein a 20 aa signal sequence, a 735 aa extracellular domain (ECD), a 21 aa transmembrane region and a 261 aa cytoplasmic region are contained. Pearls express in all mammals and have high similarity. For example, the similarity between human Pearl and mouse Pearl (Gene ID II: 73182) is 84%.

While the inventors firstly found that in addition to the expression in vascular endothelial cells as confirmed at present, Pearl is also expressed in fibroblasts, and has a negative regulation effect on activation of fibroblasts. The present disclosure uses Pearl agonists in combination with pirfenidone or nintedanib to detect the inhibitory effect on collagen synthesis of fibroblasts. The results show that the inhibition of collagen synthesis in IPF fibroblasts by using Pearl agonists in combination with pirfenidone or nintedanib is more significant than that of any drug used individually. The present disclosure uses Pearl agonists in combination with pirfenidone or nintedanib to detect the inhibitory effect on collagen synthesis of fibroblasts. The results show that the inhibition of collagen synthesis in IPF fibroblasts by using Pearl agonists in combination with pirfenidone or nintedanib is more significant than that of any drug used individually. Animal experiments also support this result.

As used herein, the terms "agonist", "Pearl agonist" or "agonist of Pearl gene or a protein thereof" may be used interchangeably to refer to substances capable of enhancing the activity of Pearl gene or protein thereof, and/or binding Pearl protein, and/or promoting Pearl to inhibit signaling pathways of fibroblast activation signaling pathway, and/or reducing ECM released from fibroblasts. Besides the known Pearl agonist, the present disclosure provides a novel antibody that specifically binds to Pearl, used as an active component in the combined drug regimen of the present disclosure.

As used herein, the term "agonist" also comprises up-regulators, acting by promoting the expression of Pearl gene. When more Pearl protein is expressed (multiply expressed), it usually means the activity of Pearl increased.

In the present disclosure, any substance that can increase the activity of Pearl, increase the stability of Pearl, up-regulate the expression of Pearl, promote the secretion of Pearl and increase the effective time of Pearl can be used in the present disclosure as useful substances for up-regulating Pearl. All of these can be called as "agonists" or "up-regulators".

As an embodiment of the present disclosure, the Pearl up-regulator comprises (but not limited to): an expression vector or an expression construct capable of expressing (preferably over-expressing) Pearl after being transferred into cells. Usually, the expression vector comprises a gene cassette, and the gene cassette comprises the gene encoding Pearl and the regulatory sequences operatively linked thereto. The term "operably linked" or "operably linked to" refers to a condition that certain parts of a linear DNA sequence can regulate or control the activity of other parts of the same linear DNA sequence. For example, a promoter is operably linked to a coding sequence if it controls the transcription of the sequence.

In the present disclosure, the Pearl polynucleotide sequence can be inserted into a recombinant expression vector, so that it can be transferred into cells and over-expressed to produce Pearl. Any plasmid and vector can be used as long as it can replicate and be stable in the hosts. An important characteristic of an expression vector is that it usually contains an origin of replication, a promoter, a marker gene, and translation control elements. For example, the expression vectors comprise: viral vectors, non-viral vectors and so on.

Preferably, the agonist provided by the present disclosure is an agonist which is capable of binding to a Pearl gene or a protein thereof and resulting a structure change of Pearl, thereby inhibiting fibroblast activation. Preferably, said agonist is an agonist that targets and binds to Pearl protein expressed on fibroblasts.

In a preferred embodiment, in addition to the antibody of the present disclosure, the agonist of the present disclosure may further comprise a substance that has a function of activating Pearl, such as FcεR1α (i.e., Fc fragment of IgE receptor Ia), dextran sulfate, fucose, or a combination thereof, and it may also comprise natural aptamer to Pearl and/or small molecule compound, more preferably, FcεR1α, dextran sulfate and fucose, with abilities of significantly inhibiting ECM synthesized by lung fibroblasts, improving lung function and increasing survival rate.

By using Pearl as a target of the present disclosure, a person skilled in the art would be able to design a variety of different agonists targeting Pearl, including, but not limited to, small molecule agonists, antibodies or active fragments thereof, miRNAs, and so on. In the present disclosure, the small molecule compound may also refer to a naturally occurred or artificially developed small molecule agonist to Pearl receptor.

In the present disclosure, the aptamer (Aptamer) refers to an in vitro screening technology: systematic evolution of ligands by exponential enrichment (Systematic evolution of ligands by exponential enrichment, SELEX). The obtained structured oligonucleotide sequence (RNA or DNA) has high recognition capability and high affinity with corresponding target molecules (proteins, viruses, bacteria, cells, heavy metal ions and so on).

As a preferred embodiment of the present disclosure, an antibody or variant thereof that specifically binds to/targets the Pearl protein is used, and the antibody or variant thereof has been confirmed to have a definite activation of Pearl at the level of cytology and zoology. Preferably, the antibodies or variants thereof of the disclosure are monoclonal antibodies derived from mice, and could be recombinant, polyclonal, multi-chained or single-chained, or intact immunoglobulin. Preferably, the antibodies or variants thereof in the present disclosure are humanized. Preferably, the antibodies of the present disclosure are chimeric antibodies. Preferably, the antibodies or variants thereof in the present disclosure are shown in Tables 1 to 4.

The antibody targeting Pearl comprises a full-length antibody and an antibody fragment (such as Fab', Fab', F(ab')₂, Fv such as scFv), and also comprise a bispecific antibody, a multispecific antibody, a single domain antibody or a monoclonal antibody or polyclonal antibody prepared from the antibody. The Fab fragment of the antibody is also called a fragment of antigen binding (Fragment of antigen binding), and refers to the domain in an antibody structure that binds to an antigen. Fab fragment consists of a complete light chain and VH and CH1 domain of a heavy chain. Under the action of papain, the antibody can be degraded into two Fab fragments and a Fc fragment. Under the action of papain, the antibody can be degraded into a (Fab)2 fragment and a Fc fragment.

FR fragments of Fab which can be used in the antibody or the variant thereof in the present disclosure are not particularly limited, preferably, FR of human IgG4 and many mutated FR sequences can be used in the present disclosure. The result shows that changing in FR sequence barely affects the binding between the antigen and the antibody. It could be seen that the CDR fragments of the antibody of the present disclosure has an excellent ability to specifically and stably bind to Pearl protein. A person skilled in the art would have been able to use the antibodies or variants thereof in the present disclosure according to the teaching of Pearl as a target of the present disclosure and the relevance to fibrotic diseases thereof, and use a plurality of currently known FR to screen the constructed antibodies, thereby obtaining an antibody having consistent or substantially consistent activity with the antibodies or the variants thereof in the present disclosure. It may also be included in the scope of the present disclosure.

Constant regions that can be used in the antibodies or variants thereof of the present disclosure are not particularly limited. A person skilled in the art would have been able to screen a constant region fragment suitable for an antibody containing the antibody or a variant thereof according to the teaching of Pearl as a target of the present disclosure and the relevance to fibrotic diseases thereof, thereby obtaining a complete antibody having consistent or substantially consistent activity with the antibodies or variants of the present disclosure.

### Pirfenidone or nintedanib

At the end of 2014, the U.S. FDA sequentially approved pirfenidone and nintedanib as orphan drugs to treat IPF, wherein pirfenidone is a multi-effective pyridine small molecule compound with characteristics of anti-inflammatory, anti-fibrosis and anti-oxidation. The pirfenidone can significantly decrease the descending rate of forced exhalation vital capacity. After continuous administration of pirfenidone for 52 weeks, the decrease of the lung function index of the patient can be slowed down, the Progression-Free Survival (PFS) of the patient is prolonged, and meanwhile, the risk of death of the patient is reduced, but the side effects comprise photosensitivity, weakness, rash, stomach discomfort and anorexia.

Nintedanib is a multi-targeted tyrosine kinase small molecule inhibitor. It can inhibit platelet-derived growth factor receptor (PDGFR), vascular endothelial growth factor receptor (VEGFR) and fibroblast growth factor receptor (FGFR). Nintedanib can significantly reduce the absolute value of FVC drop of IPF patient and relieve the disease process to a certain extent. Although both nintedanib and the pirfenidone can reduce the deterioration speed of the lung function and improve the life quality of patients, they are only suitable for delaying the descending speed of the lung function of patients, without improving the symptoms of the respiratory system. They are only applicable to the IPF patients with mild to moderate pulmonary dysfunction, and cannot be used in severe IPF patients. In addition, pirfenidone cannot be used in patients with renal dysfunction, and nintedanib cannot be used in patients with liver dysfunction, which further limited the application of these two drugs.

The structural formula of pirfenidone is as follows on the left; the structural formula of nintedanib is as follows on the right:

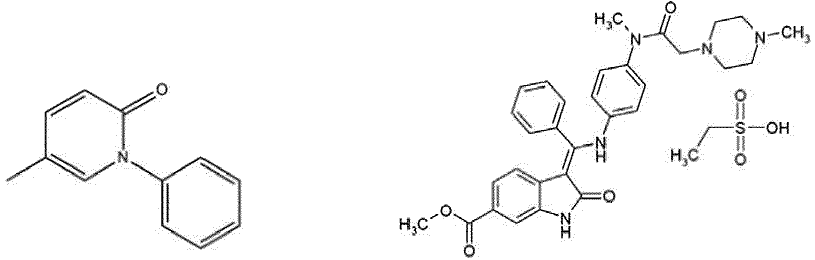

In the present disclosure, the nintedanib or pirfenidone may be a compound in pure form, or a compound with a purity greater than 85% (preferably greater than 90%, such as greater than 95%, 98%, 99%). In the case of knowing its chemical structure, it can be prepared by chemical synthesis. The nintedanib or pirfenidone can also be obtained commercially.

The present disclosure also comprises the precursor of nintedanib or pirfenidone. The "precursor" refers to a compound that, when administered by an appropriate method, undergoes metabolism or chemical reaction within the patient's body to transform into the active form of the compound.

The present disclosure also comprises the isomers, solvates, or pharmaceutically acceptable salts of the above nintedanib or pirfenidone, as long as they also have the same or substantially the same properties as nintedanib or pirfenidone. The "pharmaceutically acceptable salt" refers to the salt formed by a reaction of the compound with inorganic acid, organic acid, alkali metal or alkaline earth metal, etc. These salts comprise (but are not limited to): (1) salts formed with inorganic acids such as hydrochloric acid, sulfuric acid, nitric acid, phosphoric acid; (2) salts formed with organic acids such as acetic acid, oxalic acid, succinic acid, tartaric acid, methanesulfonic acid, maleic acid, or arginine. Other salts comprise those formed with alkali or alkaline earth metals (such as sodium, potassium, calcium or magnesium), in the form of esters, carbamates, or other conventional "prodrugs". Compounds possess one or more asymmetric centers. Thus, these compounds may exist as racemic mixtures, individual enantiomers, individual diastereoisomers, diastereomeric mixtures, cis or trans isomers.

The term "solvate" means a compound carrying a solvent molecule, for example, the solvate may be a hydrate.

The present disclosure further comprises compounds (derivatives) with the core structure based on the parent structure of nintedanib or pirfenidone, formed by appropriate group substitution. For example, on the benzene ring or multi-ring (five-membered ring/six-membered ring) of the parent structure of nintedanib or pirfenidone, substitutions such as hydrogen, hydroxy, C1-C4 alkyl (such as methyl, ethyl, n-propyl, isopropyl, n-butyl, isobutyl, tert-butyl), C2-C4 alkenyl (including straight-chain and branched hydrocarbon radicals with at least one carbon-carbon double bond and 2-4 carbon atoms, preferably 2-3 carbon atoms), C2-C4 alkynyl (including straight-chain and branched hydrocarbon radicals with at least one carbon-carbon triple bond and 2-4 carbon atoms, preferably 2-3 carbon atoms), halogens (F, Cl, Br, or I), etc., are replaced, thereby forming compounds that retain the same or similar activity as nintedanib or pirfenidone. The compounds thus obtained are also included in the present disclosure.

Under different conditions, the compound may form different crystal forms, and the present disclosure also comprises active compounds of different crystal forms.

In the present disclosure, different formulations of nintedanib and pirfenidone are also included, including oral, intravenous and inhalation formulations.

### Combination of Pearl agonist with nintedanib or pirfenidone

Although both nintedanib and pirfenidone can reduce the deterioration speed of the lung function and improve the life quality of patients, they are only suitable for delaying the descending speed of the lung function of patients, without improving the symptoms of the respiratory system. They are only applicable to the IPF patients with mild to moderate pulmonary dysfunction, and cannot be used in severe IPF patients. In addition, pirfenidone cannot be used in patients with renal dysfunction, and nintedanib cannot be used in patients with liver dysfunction, which further limited the application of these two drugs.

The present disclosure provides a method using a pharmaceutical combination, comprising administering an agonist targeting Pearl in combination with nintedanib (including its derivatives) or pirfenidone (including its derivatives). Compared with the single use of nintedanib or pirfenidone, the pharmaceutical combination in the present disclosure can effectively reduce the dosage of the compound, reduce its toxic and side effects, and effectively improve the therapeutic effect.

As a preferred embodiment of the present disclosure, the Pearl-targeting agonist is an anti-Pearl antibody that specifically activates Pearl.

In the art, it is known that pirfenidone or nintedanib can relieve the progress of fibrotic diseases, but their effects on alleviating or treating diseases still need to be further improved. After extensive researches and screening, the inventors found that an agonist targeting Pearl in combination with pirfenidone or nintedanib has an extremely excellent effect on alleviating or treating fibrotic diseases. Experimental results of cells by the inventors showed that the combined use exerts a stronger inhibitory effect on the activation of fibroblasts, a more effective inhibitory effect on collagen synthesis by fibroblasts and a more effective inhibitory effect on extracellular matrix protein expressed by fibroblasts than individual use of pirfenidone or nintedanib.

On this basis, the present disclosure provides a use of a pharmaceutical combination of Pearl agonist with nintedanib or pirfenidone for preparing a composition, a pharmaceutical composition or a drug kit for alleviating or treating fibrotic diseases.

During administration, an agonist targeting Pearl can be used first to regulate the expression or activity of Pearl, and then pirfenidone or nintedanib is administered; or both can be administered simultaneously. It is understood that various administration methods are contemplated by the present disclosure.

As a preferred embodiment, the present disclosure provides a composition comprising an antibody that specifically activates Pearl and nintedanib as active components. Preferably, in the composition, the mass ratio of the two is 1:(1~500), preferably 1:(1~300), more preferably 1:(3~200).

As a preferred embodiment, the present disclosure provides a composition comprising an antibody that specifically activates Pearl and pirfenidone as active components. Preferably, in the composition, the mass ratio of the two is 1:(5~2000), preferably 1:(5~1000), preferably 1:(5~500), more preferably 1:(10~300).

The present disclosure also provides a pharmaceutical composition, comprising: (a) an effective amount of an agonist targeting Pear1; (b) an effective amount of nintedanib or a derivative thereof, or pirfenidone or a derivative thereof, or a combination thereof; and (c) a pharmaceutically acceptable carrier or excipient. Usually, in the pharmaceutical composition of the present disclosure, each active ingredient can account for 0.01-20% of the total weight of the pharmaceutical composition, and the rest can be a pharmaceutically acceptable carrier. In the pharmaceutical composition of the present disclosure, effective amount of each active component described above may vary with the method of administration and the severity of the disease to be treated. When necessary, it can also be administered in combination with other active ingredients or drugs.

The pharmaceutical composition of the present disclosure may also comprise FcεR1α, dextran sulfate, fucoidan, aptamers or other small molecule compounds.

The pharmaceutically acceptable carrier of the present disclosure can be used to maintain the stability of the structure and function of the agonist of the present disclosure and facilitate administration. Those skilled in the art can select and match carriers according to their needs. Such carriers include (but are not limited to): Saline, buffer, dextrose, water, glycerol, ethanol, and combinations thereof.

The agonists, antibodies, and variants thereof of the present disclosure, and drugs and/or pharmaceutical compositions containing them, can be administered (administered) to the desired subject through a variety of routes, including but not limited to: inhalation, topical administration, parenteral administration, oral administration, enteral administration. In a preferred embodiment, those skilled in the art can determine the type of formulation to be administered according to the route of administration, for example, including a formulation by inhalation, a formulation by topical administration, a formulation by parenteral administration or a combination thereof. The formulation by inhalation includes aerosols, sprays, nasal drops, powders or combinations thereof. The formulation by topical administration and/or the formulation by parenteral administration include(s) drops, gels, emulsions, ointments, patches, films, injections or combinations thereof. In a preferred embodiment, the drug can be administered by various routes such as local coating, instillation, intravascular injection, local injection and so on.

In a more preferred embodiment, the Pearl agonist is an antibody that specifically activates Pearl and it is administered parenterally, preferably locally (for example, when inhibiting pulmonary fibrosis, it is administered in a form of spray); the nintedanib or pirfenidone is administered orally or inhaled by dry powder or atomization. The appropriate selection of administration methods and the preparation of pharmaceutical compositions with suitable formulations contribute to achieving relatively desirable therapeutic effects or effectively alleviating the toxic side effects of drugs on the body.

The agonists containing Pearl, nintedanib, or pirfenidone of the present disclosure can be stored separately or in a mixed form in suitable sterilized devices suitable for spraying, injection or infusion.

In the specific examples of the present disclosure, some dosing regimens for animals such as mice are given. It is easy for those skilled in the art to convert the dosage of animals such as mice into dosages suitable for humans. For example, it can be calculated according to the Meeh-Rubner formula: A=k×(W^{2/3})/10,000.

In the formula, A is the body surface area, calculated in m²; W is the body weight, calculated in g; K is a constant, which varies with animal species. Generally speaking, mice and rats are 9.1, guinea pigs are 9.8, rabbits are 10.1, cats are 9.9, dogs are 11.2, monkeys are 11.8, human is 10.6. It will be understood that, depending on the drug and the clinical situation, the conversion of the administered dose may vary according to the assessment of an experienced pharmacist.

The present disclosure also provides a drug kit for treating tumors, wherein the drug kit comprises: container 1/package 1, and the agonist of Pearl placed therein; and container 2/package 2 and the nintedanib (including its derivatives) or pirfenidone (including its derivatives) placed therein. The container/package may be a device suitable for administration, such as a spray device, an injective device, a capsule, and so on.

In addition, there may also be some auxiliary materials for medication in the drug kit, such as syringes for injections, sprayers and so on.

In addition, the drug kit can also comprise instructions for use, illustrating the method of inhibiting fibrotic diseases by using the method of pharmaceutical combination in the present disclosure.

The technical solution of the present disclosure is a combination of a Pearl agonist and nintedanib or pirfenidone. Although in clinical practice, the actual single-drug effect of nintedanib or pirfenidone needs to be improved, the combined application of the two found in the present disclosure has an excellent therapeutic effect, which greatly exceeds the expectations of ordinary people/physicians in this field.

The disclosure if further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual or followed the manufacturer's recommendation.

### Materials and methods

### 1. Preparation of the antibodies

Anti-human Pearl monoclonal antibodies LF2 and the like were prepared by the present inventors by using human Pearl extracellular protein to immunize BALB/c mice. High-affinity monoclonal hybridoma cells were prepared through standard mouse spleen cells and mouse myeloma cell fusion technology, and then obtained by standard antibody sequencing, 293 S cell expression, and purification.

The light chain variable region and heavy chain variable region sequences of anti-human Pearl monoclonal antibodies LF2, LF3, LF7, LF11, LF15 and LF16 are listed in Table 1, and their CDR sequences are shown in Table 2.

**Table 1**

| Antibo dy | heavy chain variable region (VH) sequence | light chain variable region (VL) sequence |
|---|---|---|
| LF2 | SEQ ID NO: 1 | SEQ ID NO: 16 |
| LF3 | SEQ ID NO: 2 | SEQ ID NO: 16 |
| LF7 | SEQ ID NO: 3 | SEQ ID NO: 17 |
| LF11 | SEQ ID NO: 2 | SEQ ID NO: 18 |
| LF15 | SEQ ID NO: 4 | SEQ ID NO: 19 |
| LF16 | SEQ ID NO: 5 | SEQ ID NO: 20 |

**Table 2**

| Antibody | SEQ ID NO: (HCDR1) | SEQ ID NO: (HCDR2) | SEQ ID NO: (HCDR3) |
|---|---|---|---|
| LF2 | 26 | 27 | 28 |
| LF3 | 26 | 27 | 29 |
| LF7 | 26 | 30 | 28 |
| LF11 | 26 | 27 | 29 |
| LF15 | 31 | 27 | 32 |
| LF16 | 33 | 34 | 35 |

| Antibody | SEQ ID NO: (LCDR1) | SEQ ID NO: (LCDR2) | SEQ ID NO: (LCDR3) |
|---|---|---|---|
| LF2 | 45 | GAT | 46 |
| LF3 | 45 | GAT | 46 |
| LF7 | 47 | SAS | 48 |
| LF11 | 49 | DTS | 50 |
| LF15 | 51 | GAT | 52 |
| LF16 | 45 | GAT | 46 |

The method of CDR transplantation are also used in the present disclosure to carry out humanization on the above-mentioned antibodies and carry out different back mutations to the sites of the framework region. The sequences of humanized antibodies hLF101-116, hLF-N55S, hLF-N55D, hLF-N55Q, hLF-G56A and hLF-G56V are also shown in Table 3.

**Table 3. Sequences of the antibodies**

| Antibody | heavy chain variable region (VH) sequence | light chain variable region (VL) sequence |
|---|---|---|
| hLF101 | SEQ ID NO: 7 | SEQ ID NO: 22 |
| hLF102 | SEQ ID NO: 7 | SEQ ID NO: 23 |
| hLF103 | SEQ ID NO: 7 | SEQ ID NO: 24 |
| hLF104 | SEQ ID NO: 7 | SEQ ID NO: 25 |
| hLF105 | SEQ ID NO: 8 | SEQ ID NO: 22 |
| hLF106 | SEQ ID NO: 8 | SEQ ID NO: 23 |
| hLF107 | SEQ ID NO: 8 | SEQ ID NO: 24 |
| hLF108 | SEQ ID NO: 8 | SEQ ID NO: 25 |
| hLF109 | SEQ ID NO: 9 | SEQ ID NO: 22 |
| hLF110 | SEQ ID NO: 9 | SEQ ID NO: 23 |
| hLF111 | SEQ ID NO: 9 | SEQ ID NO: 24 |
| hLF112 | SEQ ID NO: 9 | SEQ ID NO: 25 |
| hLF113 | SEQ ID NO: 10 | SEQ ID NO: 22 |
| hLF114 | SEQ ID NO: 10 | SEQ ID NO: 23 |
| hLF115 | SEQ ID NO: 10 | SEQ ID NO: 24 |
| hLF116 | SEQ ID NO: 10 | SEQ ID NO: 25 |
| hLF-N55S | SEQ ID NO: 11 | SEQ ID NO: 22 |
| hLF-N55D | SEQ ID NO: 12 | SEQ ID NO: 22 |
| hLF-N55Q | SEQ ID NO: 13 | SEQ ID NO: 22 |
| hLF-G56A | SEQ ID NO: 14 | SEQ ID NO: 22 |
| hLF-G56V | SEQ ID NO: 15 | SEQ ID NO: 22 |

The CDR sequences of the antibodies of the present disclosure are shown as SEQ ID NO: 26-55 in Table 4, and the CDR sequences of these antibodies are under the IMGT numbering rules.

**Table 4. CDR sequences under the IMGT numbering rules**

| Antibody | SEQ ID NO(HCDR1) | SEQ ID NO(HCDR2) | SEQ ID NO(HCDR3) |
|---|---|---|---|
| LF2 | 26 | 27 | 28 |
| hLF101-106 | 39 | 27 | 28 |
| hLF-N55S | 39 | 40 | 28 |
| hLF-N55D | 39 | 41 | 28 |
| hLF-N55Q | 39 | 42 | 28 |
| hLF-G56A | 39 | 43 | 28 |
| hLF-G56V | 39 | 44 | 28 |

| Antibody | SEQ ID NO(LCDR1) | SEQ (LCDR2) | SEQ ID NO(LCDR3) |
|---|---|---|---|
| LF2 | 45 | GAT | 46 |
| hLF101-116 | 45 | GAT | 46 |
| hLF-N55S | 45 | GAT | 46 |
| hLF-N55D | 45 | GAT | 46 |
| hLF-N55Q | 45 | GAT | 46 |
| hLF-G56A | 45 | GAT | 46 |
| hLF-G56V | 45 | GAT | 46 |

### 2. Cell line

IPF fibroblasts of patients were purchased from the ATCC cell library.

Pirfenidone and nintedanib were purchased from Selleck.

### 3. Detection of hydroxyproline

After the treatment of starvation in cultured IPF fibroblasts for 12 hours, 1 µg/mL Pear1 agonist (anti-Pearl antibodies LF2, LF3, LF7), 0.3 mg/mL pirfenidone, 1 µM nintedanib, 1 µg/mL Pearl agonist (anti-Pearl antibodies LF2, LF3, LF7) combined with 0.3 mg/mL pirfenidone, 1 µg/mL Pearl agonist (anti-Pearl antibodies LF2, LF3, LF7) combined with 1 µM nintedanib were added separately to treat the cells. After 30 minutes, 10ng/mL TGFβ was added to treat the cells for 48 hours, then the cell supernatant was collected. The content of hydroxyproline was detected using the hydroxyproline detection kit of Nanjing Jiancheng Biology Co., Ltd. according to the instructions.

### 4. Quantitative Real-time PCR

IPF fibroblasts in Method 2 were collected. Total mRNA was extracted by Trizol method (Invitrogen, Carlsbad, CA) according to standard protocols. Complementary DNA was synthesized using reverse transcriptase (Takara). ABI7000 Real-time PCR instrument (Life Technologies) was used to detect gene expression and the PCR primer sequences are shown in Table 1. The 18sRNA gene was used as an internal reference.

**Table 5. qPCR primer sequences**

| Gene | Forward primer | Reverse primer |
|---|---|---|
| Col 7a1 | ttacgccgctgacattgtgtt(SEQ ID NO: 104) | accagcccttcgagaaagc(SEQ ID NO: 105) |
| Timp1 | ggagagtgtctgcggatacttc(SEQ ID NO: 106) | gcaggtagtgatgtgcaagagtc(SEQ ID NO: 107) |
| 18sRNA | gagcggtcggcgtcccccaacttc(SEO ID NO: 108) | cctcaccccggcatctaa(SEQ ID NO: 109) |

### 5. Construction of Pearl humanized transgenic mice

The human Pearl gene was introduced into mice by CRISPR/Cas9 technology. Target sites were designed near exon 1 and the ATG start codon of the mouse Pearl gene. With the presence of a homologous recombinant template, the exogenous human Pearl gene was precisely integrated into the designated location, replacing the mouse Pearl gene sequence.

### 6. IPF model preparation

According to the body weight, Pearl humanized transgenic mice were anesthetized by intraperitoneal injection of pentobarbital sodium (100mg/kg). After the breathing of mice being steady, intratracheal aerosolization of 0.8mg/ml bleomycin was administered at the bottom of trachea to induce pulmonary fibrosis in mice. After administration, wounds of mice were quickly sutured. The mice were put back into the cage and kept breathing unobstructed. The infrared heating lamp was used to warm the mice, waiting for the mice to wake up.

### 7. Mouse Lung Function Measurement

Mice are anesthetized by intraperitoneal injection of pentobarbital (100 mg/kg), and each mouse is connected with a computer controlled small animal breathing machine through an air pipe sleeve according to the description of the AniRes2005 lung function analysis system (BestLab version 2.0, AniRes2005, China) manufacturer. The change in pressure in the plethysmography chamber is measured by a port in a connecting tube with a pressure sensor, with a forced vital capacity (FVC) and a 0.1 second forced expiratory volume (FEV 0.1) as an indicator of the lung function.

### 8. Masson Staining and immunofluorescence staining

Mouse lung was fixed in 4% paraformaldehyde for 24 hours, after OCT embedding, a frozen slicing machine was used for slicing. Masson staining and immunofluorescence staining of pdgfra and collagen were then performed. The area of the positive region was calculated using Image J software.

### 9. Statistical methods

All data were statistically analyzed using Graphpad8.0 software. The t test was used to compare the data between the two groups. Kaplan-Meier analysis was used for survival analysis. In all data comparisons, p-values less than 0.033 were considered statistically significant.

### Example 1. Effect of Pearl agonist in combination with pirfenidone or nintedanib on collagen secretion in fibroblasts

Fibroblasts can proliferate abnormally and transform into activated fibroblasts to synthesize and secrete a large amount of extracellular matrix proteins, such as collagen, periostin, fibronectin, etc., leading to the occurrence of pulmonary fibrosis. Hydroxyproline is a collagen-specific amino acid. Therefore, experimental groups, including treatment with Pearl agonist (antibody LF2) alone, pirfenidone alone, nintedanib alone, the combination of Pearl agonist (antibody LF2) with pirfenidone, and the combination of Pearl agonist (antibody LF2) with nintedanib were set up alongside control groups. These groups were treated with TGFβ-induced IPF fibroblasts for 48 hours. Subsequently, the content of hydroxyproline in the supernatant of cultured cells was measured using an assay kit to characterize collagen secretion, reflecting the activation of fibroblasts.

The results indicate that the combination of Pearl agonist and pirfenidone, or the combination of Pearl agonist and nintedanib has a more significant inhibitory effect on collagen secretion by IPF fibroblasts compared to either drug used alone, as shown in Figure 1.

### Example 2. Effect of Pearl agonist in combination with pirfenidone or nintedanib on collagen secretion in fibroblasts

As the method used in Example 1, experimental groups, including treatment with Pearl agonist (antibody LF3 or LF7) alone, pirfenidone alone, nintedanib alone, the combination of Pearl agonist (antibody LF3 or LF7) with pirfenidone, and the combination of Pearl agonist (antibody LF3 or LF7) with nintedanib were set up alongside control groups. The content of hydroxyproline when using Pearl agonist (antibody LF3 or LF7) in combination with pirfenidone or nintedanib in IPF fibroblasts was detected to characterize collagen secretion, reflecting the activation of fibroblasts.

The results indicate that the combination of Pearl agonist and pirfenidone, or the combination of Pearl agonist and nintedanib has a more significant inhibitory effect on collagen secretion by IPF fibroblasts compared to either drug used alone, as shown in Figure 2, Figure 3.

### Example 3. Effect of Pearl agonist in combination with pirfenidone or nintedanib on extracellular matrix synthesis in fibroblasts

Experimental groups including anti-Pearl antibodies LF11, LF15, LF16 used alone, and their combinations with pirfenidone or nintedanib respectively, and experimental groups including pirfenidone or nintedanib used alone and a control group were established. TGFβ-induced IPF fibroblasts were treated for 48 hours, and then cells were collected. The qPCR method was used to detect the effect on the synthesis of extracellular matrix proteins collagen 7a1 and matrix metalloproteinase inhibitor 1 (primers are shown in Table 1).

The results indicate that Pearl agonists (antibodies LF11, LF15, LF16), pirfenidone, and nintedanib can inhibit the expression of extracellular matrix proteins in IPF fibroblasts to some extent. Among them, the combination of LF16 antibody with pirfenidone or nintedanib has a more significant inhibitory effect on the expression of extracellular matrix proteins in IPF fibroblasts compared to pirfenidone or nintedanib used alone, as shown in Figure 4A-B, Figure 5A-B, Figure 6A-B.

Above results showed that the combination of Pearl agonist with pirfenidone and nintedanib has a stronger inhibitory effect on the activation of fibroblasts than pirfenidone or nintedanib used alone.

### Example 4. Effects of Pearl agonist in combination with pirfenidone or nintedanib on bleomycin-induced pulmonary fibrosis in mice

Humanized antibodies hLF105 and hLF-G56V were aerosolized and sprayed into the lungs of Pearl humanized transgenic mice at a dose of 1 µg/g on the 7th and 14th day after modeling, respectively. Pirfenidone at a dose of 300 mg/kg was intragastrically administered once a day from the day before modeling. Nintedanib at a dose of 60 mg/kg was intragastrically administered twice a day from the 7th day after modeling. Then, on the 21st day, the lung function of the mice was analyzed, the pathological sections were stained and the collagen deposition and fibroblast proliferation were counted. The experimental groups are shown in Table 6 (n=20).

**Table 6. Experimental groups**

| Group/Drug | hLF105(ug/g) | hLF-G56V(ug/g) | Pirfenidone (mg/kg) | Nintedanib (mg/kg) |
|---|---|---|---|---|
| 1 | 1 | | 300 | / |
| 2 | 1 | | / | 60 |
| 3 | | 1 | 300 | / |
| 4 | | 1 | / | 60 |
| Single drug group A | 1 | | / | / |
| Single drug group B | / | | 300 | / |
| Single drug group C | / | | / | 60 |
| Placebo group | NS | | NS | NS |

The results showed that when hLF105 and hLF-G56V of the present disclosure were used in combination with pirfenidone or nintedanib respectively, the improvement of mouse lung function was significantly better than that of the single drug, also with significantly reduced fibroblasts and significantly reduced collagen deposition compared with the single-use group shown in pathological slices of mice (Figure 7).

### Example 5. Effects of Pearl agonist in combination with pirfenidone or nintedanib on bleomycin-induced pulmonary fibrosis in mice

Humanized antibody hLF105 and pirfenidone or nintedanib were selected to determine the improvement in pulmonary fibrosis with reduced drug dosage, including analyzing mouse lung function, conducting pathological section staining, and quantifying collagen deposition as well as fibroblast proliferation. The experimental groups are outlined in Table 7.

**Table 7. Experimental groups**

| Group/Drug | hLF105(ug/g) | Pirfenidone (mg/kg) | Nintedanib (mg/kg) |
|---|---|---|---|
| Dose group 1 | 0.5 | 150 | / |
| Dose group 2 | 0.5 | / | 30 |
| Single drug group A | 1 | / | / |
| Single drug group B | / | 300 | / |
| Single drug group C | / | / | 60 |
| Single drug group D | 0.5 | | |
| Single drug group E | | 150 | |
| Single drug group F | | | 30 |
| Placebo group | NS | NS | NS |

The results indicate that even with a simultaneous reduction in the dosage of Pearl agonist and pirfenidone or nintedanib, a similar improvement in lung function is achieved compared to the positive control group. Additionally, pathological sections show (Figure 8) a significantly lower number of fibroblasts than the control group, with collagen deposition similar to the positive control group.

Therefore, appropriately reducing the dosage of the combination of the present disclosure still achieves a relatively ideal therapeutic effect, with a significant reduction of adverse reactions in animals.

Each reference provided herein is incorporated by reference to the same extent as if each reference was individually incorporated by reference. In addition, it should be understood that based on the above teaching content of the disclosure, those skilled in the art can practice various changes or modifications to the disclosure, and these equivalent forms also fall within the scope of the appended claims.

## Claims

1. A use in the preparation of a mixture, a pharmaceutical composition or a drug kit for alleviating or treating fibrotic diseases by a pharmaceutical combination; wherein, the pharmaceutical combination comprises:
(a) a Pear1 gene, an encoded protein or an agonist thereof; and
(b) nintedanib or a derivative thereof, pirfenidone or a derivative thereof, or a combination thereof.

2. The use according to claim 1, wherein, the fibrotic diseases comprise: pulmonary fibrosis, liver fibrosis, cardiac fibrosis, renal fibrosis, myelofibrosis, scars in skin and/or scars in soft tissues.

3. The use according to claim 1, wherein, the Pearl gene, encoded protein or agonist thereof comprises an agent that increase the expression, activity, stability, and/or effective time of Pearl; preferably, it comprises: an antibody that specifically activates Pearl, an antigen-binding fragment or variant thereof, an expression construct for recombinantly expressing Pearl, a chemical agonist of Pearl, an up-regulator that promotes the driving ability by a promoter of Pearl gene; more preferably, the chemical agonist of Pearl comprise: FcεR1α, dextran sulfate, fucoidan, or combinations thereof.

4. The use according to claim 1, wherein, the mixture, pharmaceutical composition or drug kit are also used for:
inhibiting collagen synthesis by fibroblasts; and/or
inhibiting protein expression of extracellular matrix by fibroblasts.

5. A pharmaceutical composition for alleviating or treating fibrotic diseases, wherein the pharmaceutical composition comprises:
(a) a Pearl gene, an encoded protein or an agonist thereof;
(b) nintedanib or a derivative thereof, pirfenidone or a derivative thereof, or a combination thereof; and
a pharmaceutically acceptable carrier.

6. A composition for alleviating or treating fibrotic diseases, wherein the composition is composed of the following components:
(a) a Pearl gene, an encoded protein or an agonist thereof; and
(b) nintedanib or a derivative thereof, pirfenidone or a derivative thereof, or a combination thereof.

7. A drug kit for alleviating or treating fibrotic diseases, wherein the drug kit comprises a container or package, and separated or mixed in the container or package: (a) a Pearl gene, an encoded protein or an agonist thereof; and (b) nintedanib or a derivative thereof, pirfenidone or a derivative thereof, or a combination thereof; or
the dug kit comprises the pharmaceutical composition according to claim 4; or
the dug kit comprises the composition according to claim 5.

8. The use according to claim 1, the pharmaceutical composition according to claim 5, the composition according to claim 6 or the drug kit according to claim 7, wherein (a) is an antibody that specifically activates Pearl, an antigen-binding fragment or variant thereof; wherein:
the mass ratio of the content of the antibody that specifically activates Pearl, antigen-binding fragment or variant thereof in (a) and the nintedanib or derivative thereof in (b) is 1:(1~500), preferably 1:(1~300), more preferably 1:(3~200); or
the mass ratio of the content of the antibody that specifically activates Pearl, antigen-binding fragment or variant thereof in (a) and the pirfenidone or derivative thereof in (b) is 1:(5~2000), preferably 1:(5~1000), preferably 1:(5~500), more preferably 1:(10~300).

9. The use according to claim 1, the pharmaceutical composition according to claim 5, the composition according to claim 6 or the drug kit according to claim 7, wherein, the agonist is an antibody specifically activates Pearl, an antigen-binding fragment or variant thereof; preferably, the antibody comprises a heavy chain variable region and/or a light chain variable region, and the HCDR1, HCDR2 and HCDR3 amino acid sequences of the heavy chain variable region are as shown in SEQ ID NO: 26, 27 and 28, or as shown in SEQ ID NO: 26, 27 and 29, or as shown in SEQ ID NO: 26, 30 and 28, or as shown in SEQ ID NO: 31, 27 and 32, or as shown in SEQ ID NO: 33, 34 and 35; and/or the LCDR1, LCDR2 and LCDR3 amino acid sequences of the light chain variable region are as shown in SEQ ID NO: 45, GAT and SEQ ID NO: 46, or as shown in SEQ ID NO: 47, SAS and SEQ ID NO: 28, or as shown in SEQ ID NO: 49, DTS and SEQ ID NO: 50, or as shown in SEQ ID NO: 51, GAT and SEQ ID NO: 52;
more preferably, the heavy chain variable region of the antibody is as shown in SEQ ID NO: 1, 2, 3, 4, 5, 7, 8, 9, 10, 11, 12, 13, 14 or 15, or the light chain variable region of the antibody is as shown in SEQ ID NO: 16, 17, 18, 19, 20, 22, 23, 24 or 25.

10. The use according to claim 1, the pharmaceutical composition according to claim 5, the composition according to claim 6 or the drug kit according to claim 7, wherein, the formulation of component, mixture or composition in the mixture, pharmaceutical composition or drug kit may be classified:
based on physical form, comprising a formulation selected from gas formulations such as aerosols, dry powder inhalers, aerosol inhalers; liquid formulations such as syrups, injections, suspensions; solid formulations such as lyophilized agents, powders, granules, tablets; semi-solid formulations such as ointments, pastes; or combinations thereof;
based on administration routes, comprising a formulation selected from inhalation formulations for respiratory delivery, gastrointestinal formulations, rectal formulations, injectable formulations, transdermal formulations, mucosal formulations, or combinations thereof.
